# NOUVEAU FASCICULE DE BREVET EUROPEEN

(11) **EP 1 765 356 B2**
(45) Date de publication et mention de la décision concernant l'opposition: **05.01.2011**
(45) Mention de la délivrance du brevet: 28.11.2007
(21) Numéro de dépôt: 05777271.7
(22) Date de dépôt: 15.06.2005
(51) Int. Cl.: A61K 31/593, A61K 31/57, A61K 9/12, A61K 47/10, A61K 47/44, A61P 17/00, A61P 17/06

(54) **COMPOSITION SOUS FORME DE SPRAY COMPRENANT UNE ASSOCIATION DE CALCITRIOL ET DE PROPIONATE DE CLOBETASOL, UNE PHASE ALCOOLIQUE, AU MOINS UN SILICONE VOLATILE ET UNE PHASE HUILEUSE NON VOLATILE**
SPRAYZUSAMMENSETZUNG MIT EINER KOMBINATION AUS CALCITRIOL UND CLOBETASOLPROPIONAT, EINER ALKOHOLISCHEN PHASE, MINDESTENS EINER FLÜCHTIGEN SILIKONPHASE UND EINER NICHTFLÜCHTIGEN ÖLIGEN PHASE
SPRAY COMPOSITION COMPRISING A COMBINATION OF CALCITRIOL AND CLOBETASOL PROPIONATE, AN ALCOHOLIC PHASE, AT LEAST ONE VOLATILE SILICONE AND ONE NON VOLATILE OILY PHASE

(30) Priorité: 17.06.2004 FR 0406614
(43) Date de publication de la demande: 28.03.2007
(73) Titulaire: Galderma S.A., 6330 Cham (CH)
(72) Inventeur: WILLCOX, Nathalie, F-06460 Saint Vallier de Thiey (FR); ORSONI, Sandrine, F-06210 Mandelieu (FR); MALLARD, Claire, F-06250 Mougins (FR)
(74) Mandataire: Allab, Myriam
(86) Numéro de dépôt international: PCT/FR2005/001497
(87) Numéro de publication internationale: WO 2006/005845

(56) Documents cités:
- EP-A- 0 966 972
- WO-A-00/64450
- WO-A-2004/112798
- WO-A1-2005/123091
- FR-A- 2 709 131
- FR-A- 2 737 118
- FR-A- 2 740 038
- US-A- 4 610 978
- US-A- 5 972 920
- US-A1- 2003 170 194
- LAMBA S ET AL: "Combination therapy with vitamin D analogues." BRITISH JOURNAL OF DERMATOLOGY, vol. 144, no. Supplement 58, avril 2001 (2001-04), pages 27-32, XP002253887 ISSN: 0007-0963
- AUSTAD J ET AL: "Clobetasol propionate followed by calcipotriol is superior to calcipotriol alone in topical treatment of psoriasis." JOURNAL OF THE EUROPEAN ACADEMY OF DERMATOLOGY AND VENEREOLOGY: JEADV. NETHERLANDS JUL 1998, vol. 11, no. 1, juillet 1998 (1998-07), pages 19-24, XP001154650 ISSN: 0926-9959

## Description

L'invention se rapporte à une composition anhydre sous forme de spray comprenant en tant qu'actif pharmaceutique l'association de propionate de clobétasol et de calcitriol, une phase alcoolique, au moins un silicone volatile et une phase huileuse non volatile dans un milieu physiologiquement acceptable, à son procédé de préparation, à son utilisation en cosmétique et en dermatologie. La composition permet d'obtenir une bonne pénétration de l'actif à travers les couches cutanées.

L'association de principes actifs n'est pas utilisée d'une manière classique dans le traitement des affections dermatologiques. Les difficultés principalement rencontrées par l'homme du métier lors de l'association de deux principes actifs sont les problèmes d'instabilité chimique et les interactions que les principes actifs peuvent présenter, lorsqu'ils sont présents au sein de la même formulation.

Peu de traitement existe donc associant le calcitriol et un corticoïde. En effet, la vitamine D et ses dérivés sont instables dans les milieux aqueux, et sensibles aux pH acides alors que les corticoïdes et plus particulièrement le propionate de clobétasol, sont eux, sensibles aux milieux basiques. Il n'était donc pas évident pour l'homme du métier d'associer et de stabiliser au sein d'une même composition un actif de type vitamine D et un corticostéroïde.

Le calcitriol est un analogue de la vitamine D utilisé pour réguler le taux de calcium dans l'organisme. Son utilisation dans le traitement des maladies dermatologiques a notamment été décrite dans le brevet US 4,610,978 pour le traitement du psoriasis. Ce brevet suggère des compositions comprenant du calcitriol qui peuvent en outre contenir une quantité d'un agent anti-inflammatoire tel qu'un corticostéroïde, cependant aucun mode de réalisation concret d'association de calcitriol et de corticostéroide n'est décrit ni testé en terme d'efficacité.

La demanderesse a décrit dans la demande FR 2 848 454 que l'association du calcitriol avec un corticostéroïde permet d'obtenir un effet synergique dans le traitement de certaines affections dermatologiques telles que le psoriasis, la dermatite atopique, la dermatite de contact et la dermatite séborrhéique, sans toutefois proposer des compositions pharmaceutiques stables associant les deux actifs.

Par ailleurs, dans le domaine de la dermatologie et de la formulation de compositions pharmaceutiques, l'homme du métier est amené à chercher des compositions qui, non seulement doivent être stables physiquement et chimiquement mais également doivent permettre de libérer l'actif et de favoriser sa pénétration à travers les couches cutanées afin d'en améliorer son efficacité.

Les compositions pharmaceutiques doivent, de plus, présenter une bonne cosméticité et être préférentiellement non irritantes.

Il existe actuellement de nombreuses compositions topiques comprenant un agent actif et permettant de favoriser sa pénétration dans la peau grâce à la présence notamment d'une forte teneur en glycol pro-pénétrant. Ces compositions sont formulées sous formes d'émulsions à forte teneur en phase grasse que l'on appelle communément "lipocrèmes", sous formes de compositions anhydres que l'on appelle "onguents", sous forme de compositions fluides à forte teneur en solvants volatiles, tels que l'éthanol ou l'isopropanol, destinées à une application sur le cuir chevelu, appelées également "lotions capillaires", ou encore sous forme d'émulsions H/E visqueuses, que l'on appelle aussi "crèmes H/E".

La stabilisation d'une formulation comprenant un tel pourcentage de glycol rend nécessaire l'emploi dans l'émulsion d'agents émulsifiants et stabilisants de type glycéryl stéarate ou PEG 100 stéarate ou encore d'agents stabilisants ou facteurs de consistance de type cire blanche ou alcool cétostéarylique qui conduisent à la formation d'une crème visqueuse, c'est à dire dont la viscosité est supérieure à 10 Pa.s (10000 centipoises, mesurée avec un appareil Brookfield modèle LVDV II + mobile n° 4, à une vitesse de 30 tours/min pendant 30 secondes et à une température de 25 °C +/- 3 °C). Cette viscosité confère donc au produit une difficulté d'application. Ces compositions présentent donc d'une part, une mauvaise acceptabilité cosmétique due à leur viscosité, et, d'autre part, des risques d'intolérance provoqués par la présence de fortes proportions de glycol. De plus ces viscosités élevées confèrent aux formulations des difficultés d'application sur les différentes parties du corps touchées par la pathologie. En conséquence, la plupart des traitements existants, sous forme de crèmes ou gels ou pommades, nécessitent l'aide d'une tierce personne pour les appliquer sur les zones difficiles d'accès. La tierce personne doit donc toucher à la fois le produit contenant l'actif et les plaques de psoriasis, ce qui conduit à une situation non idéale d'un point de vue confort d'utilisation et sécurité de la tierce personne. L'homme du métier sait également que la non observance du traitement prescrit pour des raisons invoquées précédemment est l'une des causes principales d'échec du traitement, l'article « patients with psoriasis and their compliance with medication » (Richards & all, J Am Acad Dermatol Oct 99, p581-583) indique que près de 40% des patients avec une maladie chronique telle que le psoriasis ne suivent pas leur traitement. Il a été démontré que l'adhésion du patient à son traitement est directement liée aux caractéristiques du véhicule de la composition appliquée. L'article « Patients with psoriasis prefer solution and foam vehicles : a quantitative assessment of vehicle preference » (Housman & all ; CUTIS, dec 2002 vol 70, p 327 à 332) indique que les patients psoriatiques préfèreront une solution ou une mousse plutôt qu'un onguent, une crème ou un gel.

L'homme du métier connaît par ailleurs des formulations contenant des composés siliconés conduisant à des compositions agréables d'utilisation. Ainsi, dans le brevet US 6,538,039, une nouvelle formulation d'actif pour une administration transdermique a été mise au point comprenant des composés siliconés afin de déposer un film à la surface de la peau. Dans cette demande également, le passage transdermique est facilité par la présence obligatoire de promoteur d'absorption que sont, entre autres composés cités, les glycols.

Dans la demande de brevet EP 0 966 972, les compositions décrites peuvent être formulées sous forme de spray et comprennent un composé actif, une gomme silicone et un excipient pharmaceutiquement acceptable. Le problème que se propose de résoudre l'invention décrite dans EP 0 966 972 est de déposer un film substantif à la surface de la peau, problème résolu grâce à la présence de la gomme de silicone.

Le problème que se propose de résoudre ici la présente invention, est de concevoir une composition stable physiquement et chimiquement permettant d'associer au sein d'une même formule le calcitriol et le propionate de clobetasol (ou 17-propionate de clobetasol, ces deux dénominations étant utilisées indifféremment par la suite). La composition doit améliorer la pénétration des actifs pharmaceutiques, tout en évitant la présence de forte teneur en glycol. La composition selon l'invention doit également présenter une facilité d'utilisation et une cosméticité acceptable pour une application sur toutes les zones du corps pouvant être touchées par la pathologie.

L'art antérieur le plus proche de l'invention est la demande internationale WO 00/64450 mentionnant l'utilisation d'une composition pharmaceutique contenant un analogue de la vitamine D et un corticostéroïde. Tous les exemples de compositions de cette demande de brevet associent uniquement le calcipotriol et le dipropionate de bétaméthasone. Les compositions préférées décrites dans la demande et permettant de stabiliser les deux actifs sont des compositions sous forme d'onguent. Mais ces compositions présentent les inconvénients cités plus haut en ce qui concerne le confort et la facilité d'application. La lecture de cet art antérieur ne permet en aucun cas à l'homme du métier de déduire les compositions pulvérisables, donc faciles d'application telles que décrites dans la présente demande avec les actifs propionate de clobetasol et calcitriol solubilisés et stables au sein de la composition.

Le problème que se propose de résoudre ici la présente invention, est donc de concevoir une composition stable physiquement et chimiquement, permettant d'associer au sein d'une même composition les deux actifs calcitriol et propionate de clobétasol agissant en synergie pour le traitement du psoriasis, la composition selon l'invention devant également présenter une facilité d'utilisation et une cosméticité acceptable pour une application sur toutes les zones du corps pouvant être touchées par la pathologie.

Par stabilité physique selon l'invention, on entend une composition ne présentant aucune modification d'aspect macroscopique (séparation de phase, changement de couleur ou d'aspect, etc..) ni microscopique (recristallisation des actifs) après stockage aux températures de 4°C et 40°C, pendant 2, 4, 8, 12 semaines.

Par stabilité chimique selon l'invention, on entend une composition dans laquelle la teneur en principe actif reste stable après trois mois à température ambiante et à 40 °C. Une teneur stable en principe actif signifie selon l'invention que le teneur présente très peu de variation par rapport à la teneur initiale, c'est-à-dire que la variation de teneur en principe actif au temps T ne doit pas être inférieure à 90% de la teneur initiale T0, et préférentiellement pas inférieure à 95% de la teneur initiale à T0.

La demanderesse a trouvé de manière surprenante que la composition liquide à température ambiante et pulvérisable comprenant, dans un véhicule pharmaceutiquement acceptable :
a) entre 0,0001 et 0,1% de propionate de clobétasol sous forme de solubilisée ;
b) entre 0,00001 et 0, 1 % de calcitriol sous forme solubilisée ;
c) entre 5 et 60% d'éthanol ;
d) entre 10 et 60% d'héxaméthyldisiloxane ;
e) entre 1 et 80% d'une phase huileuse non volatile composée de triglycérides caprilique/caprique.
ladite composition étant pulvérisable, c'est-à-dire adaptée au conditionnement sous forme de spray, conduisait à une composition qui résout les problèmes posés.

La composition de la présente invention, tout en permettant une bonne pénétration des principes actifs, présente également une très bonne acceptabilité et tolérance auprès des patients, comme décrit dans les exemples de la présente invention. Il s'avère donc que la composition selon l'invention est particulièrement adaptée au traitement des affections dermatologiques et plus particulièrement bien adaptée pour le traitement du psoriasis.

L'invention concerne donc une composition liquide à température ambiante, de préférence pulvérisable, comprenant, dans un véhicule pharmaceutiquement acceptable :
a) entre 0,0001 et 0,1% de propionate de clobétasol sous forme de solubilisée ;
b) entre 0,00001 et 0, 1 % de calcitriol sous forme solubilisée ;
c) entre 5 et 60% d'éthanol ;
d) entre 10 et 60% d'héxaméthyldisiloxane ;
e) entre 1 et 80% d'une phase huileuse non volatile composée de triglycérides caprilique/caprique.

Par forme solubilisée, on entend une dispersion à l'état moléculaire dans un liquide, aucune cristallisation de l'actif n'étant visible à l'oeil nu ni même au microscope optique en polarisation croisée.

Par composition pulvérisable, on entend une composition liquide, fluide et qui s'écoule rapidement sous son propre poids, à température ambiante. Par température ambiante, on entend une température d'environ 25°C.
Le spray peut être obtenu par des moyens conventionnels de formulation connus de l'homme du métier, comme cela est expliqué ci-après.

De préférence, la composition est anhydre. Par composition anhydre, on entend au sens de la présente invention, une composition sensiblement exempte d'eau, c'est-à-dire présentant une teneur en eau inférieure ou égale à 1% en poids par rapport au poids total de la composition, en particulier inférieure ou égale à 0,5%, de préférence égale à zéro.

La composition selon l'invention comprend entre 0,00001 et 0,1 % en poids par rapport au poids total de la composition de calcitriol, de préférence entre 0,0001 et 0,001 % en poids, et plus préférentiellement entre 0,0002 et 0,0005 % en poids. La composition selon l'invention comprend plus particulièrement 0,0003 % de calcitriol en poids par rapport au poids total de la composition.

La composition selon l'invention comprend entre 0,0001 et 0,1 % en poids par rapport au poids total de la composition de propionate de clobétasol, de préférence entre 0,001 et 0,05 % en poids. Les compositions préférées selon l'invention comprennent plus particulièrement 0,025 % ou 0,05 % de propionate de clobétasol en poids par rapport au poids total de la composition.

Par phase alcoolique, on entend au moins un composé alcoolique. La composition selon l'invention contient de l'éthanol.
La composition contient de l'éthanol entre 5 et 60 % en poids par rapport au poids total de la composition, de préférence entre 10 et 40 % en poids.
Une composition préférée selon l'invention contient entre 10 et 40 % en poids d'éthanol.

Par silicone volatile, on entend des composés polyorganosiloxanes, pouvant être cycliques ou linéaires, ayant une pression mesurable dans des conditions ambiantes. Les silicones volatiles cycliques, sont les polydiméthylcyclosiloxanes, à savoir des composés de formule 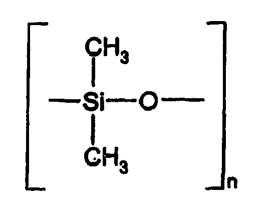 avec n compris entre 3 et 6 en moyenne et de préférence n=4 ou n=5, généralement connus sous le nom de cyclométhicones. Les silicones volatiles linéairessont des polysiloxanes linéaires tels l'héxaméthyldisiloxane ou les diméthicones de bas poids moléculaires. Les silicones volatiles linéaires ont généralement une viscosité inférieure à environ 5 centistokes à 25°celsius, alors que les silicones volatiles cycliques ont une viscosité inférieure à environ 10 centistokes à 25°celsius.

La silicone volatile selon l'invention est l'héxaméthyldisiloxane. On peut citer à titre d'exemple le produit commercialisé par la société DOW CORNING, le DC Fluid 0.65cSt.

La composition selon l'invention comprend entre 10 et 60% en poids par rapport au poids total de la composition d'héxaméthyldisiloxane, de préférence entre 20 et 50% en poids, et plus préférentiellement entre 15 et 45% en poids.

Par phase huileuse non volatile, selon l'invention, on entend une phase huileuse non volatile convenant pour une composition pharmaceutique ou cosmétique. Les huiles non volatiles ont généralement une viscosité supérieure à environ 10 centipoises à 25°C, et peuvent atteindre une viscosité allant jusqu'à 1 000 000 centipoises à 25°C.

La phase huileuse de la composition selon l'invention est composée de triglycérides caprilique/caprique, commercialisé sous le nom de Miglyol 812.

La composition selon l'invention comprendra préférentiellement le Miglyol 812. En effet, les triglycerides sont un des composants de la peau, ils font partie des lipides naturels de la peau avec les céramides, le cholestérol, les phospholipides. Ils s'intégrent dans les couches profondes de l'épiderme et compensent la perte en hydratation de la peau. La barrière protectrice de la peau est régénérée de manière spécifique et durable.
Les « Médium chain triglycerides » dont fait partie le Miglyol 812 utilisé sont composés d'acides gras caprylique (C8) et caprique (C10) dérivés de l'huile de coco ou l'huile de noyau de palme.
Ses propriétés principales sont :
- émollient de basse viscosité, augmentant l'étalement sur la peau,
- solvant d'actif lipophile, pénètre rapidement dans la peau et favorise la pénétration d'actif, et
- pas de sensation de gras à l'application, ne laisse pas de résidus gras.

Il est particulièrement recommandé d'utiliser des émollients dans le cas de pathologie telle que le psoriasis.
Les émollients aident à hydrater et adoucir les plaques de psoriasis.

Lorsque l'on applique par exemple une forte concentration en alcool ou en solvant volatile, des sensations de brûlures peuvent déranger des patients ayant une peau très irritée.

Pour compenser ces sensations d'irritation, l'utilisation de lipides en tant qu'émollients permet de restaurer la fonction normale de la barrière cutanée et de la protéger des influences extérieures.

La composition selon l'invention comprend entre 1 et 80% en poids par rapport au poids total de phase huileuse, de préférence entre 20 et 60% en poids, et plus préférentiellement entre 30 et 45% en poids.

Selon un mode préféré, la composition selon l'invention contient également des composés anti-oxydants tels que la DL α-tocophérol, le butylhydroxyanisole ou le butylhydroxytoluène, le propyl gallate, la Superoxide Dismutase, l'ubiquinol ou certains chélatants de métaux. Les anti-oxydants préférentiellement utilisés dans la composition selon l'invention sont la DL α-tocophérol, le butylhydroxyanisole et le butylhydroxytoluène.

Selon un mode préféré de composition selon l'invention, la composition comprend également une gomme de silicone. La demanderesse a, en effet, découvert de manière surprenante qu'une composition comprenant une gomme silicone dans les concentrations définies ci-après présente une pénétration plus rapide de l'actif à travers les différentes couches cutanées.

Par gommes de silicone, on entend les gommes de silicones connues par l'homme de l'art et notamment celles décrites dans la demande de brevet EP 0 966 972 incorporée ici par référence. Selon ce mode préféré de composition selon l'invention, la gomme de silicone est introduite à la concentration comprise entre 0,001 et 3% en poids, de préférence entre 0,01 et 1% en poids. Dow Corning propose un produit commercial vendu sous le nom de DC Silmogen Carrier qui se compose de 99% d'héxaméthyldisiloxane et de 1% de gomme de silicone, produit qui pourra avantageusement être utilisé dans une des compositions selon l'invention.

La composition selon l'invention peut également contenir des tensioactifs. Les tensioactifs utilisables selon l'invention sont de type tensionactif anionique tel les carboxylates, et notamment les savons, les sulfonates d'alkyle aryle, les sulfates d'alkyle éther, les sulfates d'alkyles, les sulfates d'alcool. De façon plus particulière, les anions de ces tensioactifs sont couplés à un cation tel les cations metalliques du sodium ou du potassium. Les tensioactifs préférés selon l'invention sont aussi les tensioactifs de type polysorbate et poloxamer.
De préférence, les tensioactifs utilisés selon la présente invention sont le lauryl sulfate de Sodium, le polysorbate 80 (TWEEN 80 de la société Uniqema) et le poloxamer 124 (SYNPERONIC PEL44 de la société Uniqema).

Le véhicule pharmaceutiquement acceptable selon l'invention doit être choisi de telle manière que les propriétés avantageuses attachées intrinsèquement à la présente invention ne soient pas ou substantiellement pas altérées par l'addition envisagée. Le véhicule peut être composé d'un seul excipient tel un solvant, ou d'un mélange d'excipients tels ceux utilisés pour la formulation d'une émulsion. A titre d'exemples non limitatifs d'excipients pouvant être utilisés seuls ou en mélange, on peut citer l'eau, les solvants, les diluants, tout excipient utilisable pour la formulation d'une émulsion, d'un lait, d'un gel, d'un onguent, d'une composition moussante. Ces excipients sont des composés couramment utilisés dans la formulation de composition pharmaceutique. De façon préférentielle, les excipients selon l'invention sont l'eau, les alcools, les polyols, les éthers, les esters, les aldéhydes, les cétones, les acides et alcools gras, les esters gras. Plus préférentiellement, l'excipient utilisé sera un alcool, tel l'éthanol.

La composition selon l'invention liquide à température ambiante et pulvérisable comprend, dans un véhicule pharmaceutiquement acceptable :
a) entre 0,0001 et 0,1% de propionate de clobétasol sous forme de solubilisée ;
b) entre 0,00001 et 0, 1 % de calcitriol sous forme solubilisée ;
c) entre 5 et 60% d'éthanol ;
d) entre 10 et 60% d'héxaméthyldisiloxane ;
e) entre 1 et 80% d'une phase huileuse non volatile composée de triglycérides caprilique/caprique.

Dans un mode de réalisation préféré de l'invention, la composition selon l'invention comprend dans un véhicule pharmaceutiquement acceptable :
a) entre 0,001 et 0,05 % de propionate de clobetasol ;
b) entre 0,0002 et 0,0005 % de calcitriol ;
c) entre 10 et 40 % d'éthanol ;
d) entre 15 et 45% d'héxaméthyldisiloxane ;
e) entre 30 et 45% d'une phase huileuse composée de triglycérides caprilique/caprique.

La composition préférée selon l'invention est donc une composition liquide à température ambiante et pulvérisable, comprenant, dans un véhicule pharmaceutiquement acceptable :
a) entre 0,0001 et 0,1% de propionate de clobétasol sous forme de solubilisée ;
b) entre 0,00001 et 0, 1 % de calcitriol sous forme solubilisée ;
c) entre 5 et 60% d'éthanol ;
d) entre 10 et 60% d'héxaméthyldisiloxane ;
e) entre 1 et 80% d'une phase huileuse non volatile composée de triglycérides caprilique/caprique ;
f) un anti-oxydant ;
g) une gomme de silicone.

La composition pharmaceutique selon l'invention pourra en outre contenir des additifs inertes ou des combinaisons de ces additifs, tels que :
- des agents mouillants ;
- des agents d'amélioration de la saveur ;
- des agents conservateurs tels que les esters de l'acide parahydroxybenzoïque ;
- des agents stabilisants ;
- des agents régulateurs d'humidité ;
- des agents régulateurs de pH ;
- des agents modificateurs de pression osmotique;
- des agents émulsionnants ;
- des filtres UV-A et UV-B ;
- des agents propénétrants ;
- et des polymères synthétiques.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

La composition selon l'invention est plus particulièrement destinée au traitement de la peau et des muqueuses, elle est pulvérisable et adaptée au conditionnement sous forme de spray.

Le spray peut être obtenu par des moyens conventionnels de formulation connus de l'homme du métier. Par exemple, la composition peut être pulvérisée par un pulvérisateur mécanique qui pompe la composition au sein d'un récipient, flacon ou équivalent. De même, la composition peut être propulsée au moyen d'un gaz comme cela est bien connu par l'homme de l'art. Les gaz propulseurs conventionnels tels que l'air ou les hydrocarbones sont efficaces tant qu'ils n'interfèrent pas avec la composition. La composition passe à travers une buse qui peut être dirigée directement à l'endroit désiré de l'application. La buse peut être choisie de façon à appliquer la composition sous forme d'une vaporisation ou d'un jet de gouttelette, selon les techniques connues de l'homme de l'art. Selon l'actif pharmaceutique choisi, le mécanisme de pulvérisation doit être capable de délivrer toujours la même quantité d'actif. Les mécanismes permettant de contrôler la quantité de composition à délivrer par le spray sont également connues de l'homme de l'art. Par exemple, la quantité de gaz propulseur peut être calculée de façon à propulser l'exacte quantité de produit désirée.

De préférence pour la composition selon l'invention, on utilisera un flacon vaporisateur doseur dont les caractéristiques de surface d'application et de doses sont contrôlées et reproductibles. Par exemple, le vaporisateur utilisé est constitué d'un flacon équipé d'une valve doseuse de 25µl.

La présente invention a également pour objet l'utilisation d'une composition selon l'invention pour la fabrication d'un médicament destiné au traitement :
- des affections dermatologiques avec une composante immuno-allergique inflammatoire, avec ou sans trouble de la prolifération cellulaire, notamment le psoriasis cutané, muqueux ou unguéal, le rhumatisme psoriasique, l'atopie cutanée, telle que l'eczéma,

En particulier, l'invention se rapporte à l'utilisation d'une composition telle que définie précédemment pour la fabrication d'une composition pharmaceutique destinée au traitement du psoriasis.

Dans un mode préféré d'utilisation de la composition, celle-ci contiendra 0,025% de 17-propionate de clobétasol et 0,0003% de calcitriol en présence d'éthanol et sera utilisée pour la fabrication d'un médicament destiné à traiter le psoriasis.

Dans un mode préférée de l'invention, le ratio solvant volatil / phase non volatile est optimisé de façon à conserver une évaporation rapide des solvants propice à la pénétration des actifs, mais à présenter une teneur en phase non volatile suffisante pour apporter une substantivité à la composition et permettre ainsi de maintenir plus longtemps le produit sur la peau favorisant l'émollience.
Le ratio solvant volatil /phase non volatile sera de préférence compris entre 1 et 2,5.

Les exemples suivants montrent de façon non exhaustive des exemples de formulation de la composition selon l'invention, et un exemple de référence ainsi que des résultats de stabilité chimique et physique.

### Exemple 1 de référence : Test de stabilité du calcitriol dans différentes phases huileuse et/ou alcoolique.

Des données de stabilités du calcitriol ont été générées dans divers excipients, dont l'éthanol 100, le mélange de l'éthanol 100 (75%) / Cyclomethicone 5 (25%), ou les huiles telles que le Miglyol 812 et le Cetiol SN.

### a) Stabilité du calcitriol dans l'éthanol

Solution de calcitriol 30ppm dans qsp 100% d'éthanol absolu en présence de 0.02% de BHT.
Technique de dosage par HPLC contre substance de référence.
Au temps initial (T0), on considère que la composition comprend 100% de calcitriol.

Concentration mesurée de calcitriol en % par rapport à T0 :

| Conditions de stabilité | T 1 semaine | T 2 semaines | T 3 semaines | T 4 semaines |
|---|---|---|---|---|
| -18°C | 100.9% | 100.5% | 99.5% | 99.5% |
| +4°C | 97.7% | 98.6% | 98.1% | 97.7% |
| +30°C | / | 93.4% | / | 93.0% |

Ces résultats montrent une bonne stabilité du calcitriol dans l'éthanol.

### b) Stabilité du calcitriol dans l'éthanol / cyclopentasiloxane

Solution de calcitriol 30ppm dans qsp 75% d'éthanol absolu + 25% de cyclopentasiloxane en présence de 0.02% de BHT.
Technique de dosage par HPLC contre substance de référence.
Au temps initial (T0), on considère que la composition comprend 100% de calcitriol.

Concentration mesurée de calcitriol en % par rapport à T0 :

| Conditions de stabilité | T 2 semaines | T 3 semaines | T 4 semaines |
|---|---|---|---|
| -18°C | 100.4% | 101.3% | 99.2% |
| +4°C | 99.2% | 99.6% | 97.5% |
| +30°C | 93.8% | / | 93.3% |

Ces résultats montrent une bonne stabilité du calcitriol dans le mélange éthanol / cyclopentasiloxane.

### c) Stabilité du calcitriol dans le Miglyol 812 (caprilic/capric triglycerides)

Solution de calcitriol 30ppm dans qsp 100% de Miglyol 812 en présence de 0.4% de BHT
Technique de dosage par HPLC contre substance de référence.
Au temps initial (T0), on considère que la composition comprend 100% de calcitriol.

Concentration mesurée de calcitriol en % par rapport à T0 :

| Conditions de stabilité | T 2 semaines | T 4 semaines |
|---|---|---|
| +4°C | 98.3% | 105.2% |
| TA | 95.1% | 98.0% |
| +40°C | 91% | 93.8% |

Ces résultats montrent une bonne stabilité du calcitriol dans le Miglyol 812.

### d) Stabilité du calcitriol dans le Cetiol SN (Cetearyl isononanoate)

Solution de calcitriol 30ppm dans qsp 100% de Cetiol SN (Cetearyl isononanoate) en présence de 0.4% de BHT.
Technique de dosage par HPLC contre substance de référence.
Au temps initial (T0), on considère que la composition comprend 100% de calcitriol.

Concentration mesurée de calcitriol en % par rapport à T0 :

| Conditions de stabilité | T 2 semaines | T 4 semaines |
|---|---|---|
| +4°C | 98.6% | 98.1 % |
| TA | 98.7% | 98.4% |
| +40°C | 99.0% | 98.9% |

Ces résultats montrent une bonne stabilité du calcitriol dans le Cétiol SN.

### Exemple 2 : Procédé de fabrication des compositions des exemples suivants

La fabrication des compositions selon l'invention s'effectue à température ambiante, sous sorbonne et en lumière inactinique.

Dans un flacon introduire l'antioxydant, le calcitriol et l'alcool, mettre sous agitation jusqu'à parfaite solubilisation du calcitriol.
Puis ajouter le propionate de clobetasol, continuer l'agitation jusqu'à solubilisation du propionate de clobetasol.
Lorsque les deux actifs sont parfaitement solubilisés, introduire successivement le reste des constituants de la formule.
Laisser sous agitation jusqu'à parfaite homogénéité du mélange.

### Exemple 3 de référence

| CONSTITUANTS | % |
|---|---|
| PROPANOL-2 | Qs 100 |
| DL ALPHA TOCOPHEROL | 0.05 |
| CALCITRIOL | 0.0003 |
| CLOBETASOL 17-PROPIONATE | 0.001 |
| CETEARYL ISONONANOATE | 40 |
| 1,2 PROPANEDIOL | 10 |
| DIMETHICONOL AND HEXAMETHYL DISILOXANE | 35 |

Le mode opératoire est celui précédemment décrit à l'exemple 2.
Obtention d'une solution liquide incolore.

### Exemple 4

### a) Composition

| CONSTITUANTS | % |
|---|---|
| ETHANOL ABSOLU | Qs 100 |
| BUTYLHYDROXYTOLUENE | 0.04 |
| CALCITRIOL | 0.0009 |
| CLOBETASOL 17-PROPIONATE | 0.025 |
| MEDIUM CHAIN TRIGLYCERIDES | 30 |
| DIMETHICONOL AND HEXAMETHYL DISILOXANE | 40 |

Le mode opératoire est celui précédemment décrit à l'exemple 2.
Obtention d'une solution liquide incolore.

### b) Stabilité physique de la composition

La stabilité physique des formulations est mesurée par une observation macroscopique et microscopique de la formulation à température ambiante et à 4°C après 2, 4, 8, 12 semaines. A température ambiante, l'observation macroscopique permet de garantir l'intégrité physique des produits et l'observation microscopique permet de vérifier qu'il n'y a pas recristallisation des actifs solubilisés.
A 4°C, l'observation microscopique vérifie la non-recristallisation des actifs solubilisés.

### Spécifications à T0

*Aspect macroscopique :* Solution liquide incolore
*Aspect microscopique :* Absence de cristaux de Calcitriol et de clobetasol 17-propionate.

| Temps → | T 1 M | T2M | T3M |
|---|---|---|---|
| Conditions de stabilité ↓ | | | |
| TA | Conforme aux spécifications | Conforme aux spécifications | Conforme aux spécifications |
| +4°C | Conforme aux spécifications | Conforme aux spécifications | Conforme aux spécifications |

### c) Stabilité chimique de la composition:

### - Stabilité du calcitriol :

Dosage de l'actif par étalonnage externe en HPLC.

| Temps→ | T0 | T1M | T2M | T3M |
|---|---|---|---|---|
| Conditions de stabilité ↓ | | | | |
| TA | 104.2 | 105.9% | 105% | 105.3% |

### - Stabilité du 17-propionate de clobetasol :

Dosage de l'actif par étalonnage interne en HPLC

| Temps→ | T0 | T1M | T2M | T3M |
|---|---|---|---|---|
| Conditions de stabilité ↓ | | | | |
| TA | 98.8% | 102% | 100.20% | 98% |

### Exemple 5

### a) Composition :

| CONSTITUANTS | % |
|---|---|
| ETHANOL ABSOLU | Qs 100 |
| BUTYLHYDROXYTOLUENE | 0.04 |
| CALCITRIOL | 0.0009 |
| CLOBETASOL 17-PROPIONATE | 0.025 |
| MEDIUM CHAIN TRIGLYCERIDES | 45 |
| DIMETHICONOL AND HEXAMETHYL DISILOXANE | 25 |

Même mode opératoire que précédemment décrit à l'exemple 2.
Obtention d'une solution liquide incolore.

### b) Stabilité physique de la composition :

### Spécifications à T0

*Aspect macroscopique :* Solution liquide incolore
*Aspect microscopique :* Absence de cristaux de calcitriol et de clobetasol 17-propionate.

| Temps→ | T1M | T2M | T3M |
|---|---|---|---|
| Conditions de stabilité ↓ | | | |
| TA | Conforme aux spécifications | Conforme aux spécifications | Conforme aux spécifications |
| +4°C | Conforme aux spécifications | Conforme aux spécifications | Conforme aux spécifications |

### c) Stabilité chimique de la composition:

### Stabilité du calcitriol

### Dosage de l'actif par étalonnage externe en HPLC

| Temps → | T0 | T1M | T2M | T3M |
|---|---|---|---|---|
| Conditions de stabilité ↓ | | | | |
| TA | 103.6% | 104.8% | 103.5% | 106.30% |

### Stabilité du 17-propionate de clobetasol

### Dosage de l'actif par étalonnage interne en HPLC.

| Temps → | T0 | T1M | T2M | T3M |
|---|---|---|---|---|
| Conditions de stabilité ↓ | | | | |
| TA | 108.1% | 111% | 108.8% | 106.20% |

### Exemple 6

### a) Composition :

| CONSTITUANTS | % |
|---|---|
| ETHANOL ABSOLU | Qs 100 |
| BUTYLHYDROXYTOLUENE | 0.04 |
| CALCITRIOL | 0.0003 |
| CLOBETASOL 17-PROPIONATE | 0.025 |
| MEDIUM CHAIN TRIGLYCERIDES | 45 |
| DIMETHICONOL AND HEXAMETHYL DISILOXANE | 25 |

Même mode opératoire que précédemment décrit à l'exemple 2.

### Exemple 7

| CONSTITUANTS | % |
|---|---|
| ETHANOL ABSOLU | QS 100 |
| MEDIUM CHAIN TRIGLYCERIDE | 37 |
| BUTYLHYDROXYTOLUENE | 0.04 |
| CALCITRIOL | 0.0003 |
| CLOBETASOL 17-PROPIONATE | 0.025 |
| DIMETHICONOL AND HEXAMETHYL DISILOXANE | 40 |

Le mode opératoire est celui décrit en exemple 2
On obtient une solution liquide incolore.

### Exemple 8 : stabilité physique de la composition selon exemple 7

La stabilité des formulations est mesurée par une observation macroscopique et microscopique de la formulation à température ambiante, à 4°C et à 40°C après 2, 4, 8, 12 semaines.

A température ambiante, l'observation macroscopique permet de garantir l'intégrité physique des produits et l'observation microscopique permet de vérifier qu'il n'y a pas de recristallisation de l'actif solubilisé.
A 4°C, l'observation microscopique vérifie la non recristallisation des actifs solubilisés.
A 40°C, l'observation macroscopique vérifie l'intégrité du produit fini.

### Spécifications à T0

*Aspect macroscopique :* spray liquide incolore ou très légèrement jaune.
*Aspect microscopique:* Absence de cristaux de Calcitriol et de Clobetasol 17-propionate

| Temps→ | T 1M | T2M | T3M |
|---|---|---|---|
| Conditions de stabilité ↓ | | | |
| TA | Conforme aux spécifications | Conforme aux spécifications | Conforme aux spécifications |
| +4°C | Conforme aux spécifications | Conforme aux spécifications | Conforme aux spécifications |

### Exemple 9 : stabilité chimique des actifs au sein de la composition selon exemple 7

### Stabilité du calcitriol

| Temps→ | T0 | T1M | T2M | T3M |
|---|---|---|---|---|
| Conditions de stabilité ↓ | | | | |
| TA | 101.2% | 106.4% | 107.6% | 107.4% |

### Stabilité du 17-propionate de clobetasol

| Temps→ | T0 | T1M | T2M | T3M |
|---|---|---|---|---|
| Conditions de stabilité ↓ | | | | |
| TA | 96.4% | 97.1% | 98.4% | 97.9% |

### Exemple 10

| CONSTITUANTS | % |
|---|---|
| ETHANOL ABSOLU | QS 100 |
| MEDIUM CHAIN TRIGLYCERIDE | 17 |
| BUTYLHYDROXYTOLUENE | 0.04 |
| CALCITRIOL | 0.0003 |
| CLOBETASOL 17-PROPIONATE | 0.025 |
| DIMETHICONOL AND HEXAMETHYL DISILOXANE | 70 |

Le mode opératoire est celui décrit en exemple 2
On obtient une solution liquide incolore.

### Exemple 11

| CONSTITUANTS | % |
|---|---|
| ETHANOL ABSOLU | QS 100 |
| MEDIUM CHAIN TRIGLYCERIDE | 17 |
| BUTYLHYDROXYTOLUENE | 0.04 |
| CALCITRIOL | 0.0003 |
| CLOBETASOL 17-PROPIONATE | 0.01 |
| DIMETHICONOL AND HEXAMETHYL DISILOXANE | 70 |

Le mode opératoire est celui décrit en exemple 2
On obtient une solution liquide incolore.

### Exemple 12

| CONSTITUANTS | % |
|---|---|
| ETHANOL ABSOLU | QS 100 |
| MEDIUM CHAIN TRIGLYCERIDE | 17 |
| BUTYLHYDROXYTOLUENE | 0.04 |
| CALCITRIOL | 0.0003 |
| CLOBETASOL 17-PROPIONATE | 0.05 |
| DIMETHICONOL AND HEXAMETHYL DISILOXANE | 70 |

Le mode opératoire est celui décrit en exemple 2
On obtient une solution liquide incolore.

### Exemple 13 : Etude de la libération / pénétration in vitro sur peau humaine de l'actif 17-propionate de clobétasol contenu dans 3 formulations différentes dont une selon l'invention.

L'objectif est de quantifier la pénétration cutanée de l'actif formulé dans différentes formulations *in vitro* sur peau humaine après 16 heures d'application.

### Formulations testées:

- Crème émolliente Temovate^{®} à 0.05 % (w/w) de 17-propionate de clobétasol
- Crème Temovate^{®} à 0.05 % (w/w) de 17-propionate de clobétasol
- Composition selon l'invention de formule suivante A :

| CONSTITUANTS | % |
|---|---|
| ETHANOL ABSOLU | QS 100 |
| MEDIUM CHAIN TRIGLYCERIDE | 17 |
| BUTYLHYDROXYTOLUENE | 0.04 |
| CALCITRIOL | 0.0003 |
| CLOBETASOL 17-PROPIONATE | 0.05 |
| DIMETHICONOL AND HEXAMETHYL DISILOXANE | 70 |

Les crèmes Temovate^{®} sont commercialisées par la société GLAXOSMITHKLINE.

**Conditions expérimentales :** L'absorption percutanée est évaluée grâce à des cellules de diffusion constituées de 2 compartiments séparés par la peau humaine. Les formulations ont été appliquées sans occlusion pendant un temps d'application de 16 heures. Les formulations ont été appliquées à raison de 10 mg de formulation par cm² (*i.e.* 10 microgrammes de clobétasol 17-propionate). Pendant la durée de l'étude, le derme est en contact avec un liquide récepteur non renouvelé en fonction du temps (mode statique). Les expériences ont été réalisées avec 3 échantillons de peau provenant de 3 donneurs différents. A la fin de la période d'application, l'excès de surface est enlevé et la distribution du 17-propionate de clobétasol est quantifiée dans les différents compartiments de la peau et dans le liquide récepteur. Les concentrations de 17-propionate de clobétasol ont été quantifiées en utilisant un méthode d'HPLC/MS/MS classiquement connu de l'homme de l'art. (LQ: 1 ng.mL⁻¹).

Les résultats sont exprimés en % de la dose appliquée (moyenne +/- écart-type) et sont consignés dans le tableau ci-dessous.

| Formulation | | **Quantité totale ayant pénétré** |
|---|---|---|
| Temovate crème émolliente | Mean | **5.00** |
| | SEM | **1.34** |
| Temovate crème | Mean | **8.43** |
| | SEM | **0.79** |
| Composition A | Mean | **13.03** |
| | SEM | **5.80** |

Les résultas montrent que la quantité de clobétasol ayant pénétré avec la composition selon l'invention est supérieure à celle ayant pénétré avec les crèmes Temovate.

## Revendications

1. Composition comprenant, dans un véhicule pharmaceutiquement acceptable:
a) entre 0,0001 et 0,1 % de propionate de clobétasol sous forme solubilisée;
b) entre 0,00001 et 0,1 % de calcitriol sous forme solubilisée;
c) entre 5 et 60% d'éthanol;
d) entre 10 et 60 % d'hexaméthyldisiloxane ;
e) entre 1 et 80 % d'une phase huileuse non volatile composée de triglycérides caprilique/caprique; la composition étant liquide a température ambiante et pulvérisable.

2. Composition selon la revendication 1 comprenant, dans un véhicule pharmaceutiquement acceptable:
a) entre 0,001 et 0,05 % de propionate de clobétasol;
b) entre 0,0002 et 0,0005 % de calcitriol;
c) entre 10 et 40 % d'éthanol;
d) entre 15 et 45% d'hexamethyldisiloxane;
e) entre 30 et 45% d'une phase huileuse composée de triglycérides caprilique/caprique.

3. Composition selon l'une quelconque des revendications 1 à 2, **caractérisée en ce qu'**elle comprend également un composé anti-oxydant.

4. Composition selon l'une quelconque des revendications 1 à 3, **caractérisée en ce que** l'anti-oxydant est choisi parmi le DL α-tocopherol, le butylhydroxyanisole et le butylhydroxytoluène.

5. Composition selon l'une quelconque des revendications 1 à 4, **caractérisée en ce qu'**elle comprend également une gomme de silicone.

6. Composition selon l'une quelconque des revendications 1 à 5, **caractérisée en ce qu'**elle comprend également un composé tensioactif.

7. Composition selon la revendication 6, **caractérisée en ce que** le tensioactif est choisi parmi le lauryl sulfate de sodium, les poloxamers et les polysorbates.

8. Composition selon l'une des revendications 1 à 7, **caractérisée en ce qu'**elle comprend un agent propénétrant.

9. Utilisation d'une composition selon l'une quelconque des revendications 1 à 8 pour la fabrication d'un médicament destiné au traitement:
- des affections dermatologiques avec une composante immuno-allergique inflammatoire, avec ou sans trouble de la prolifération cellulaire, notamment le psoriasis cutané, muqueux ou ungueal, le rhumatisme psoriasique, l'atopie cutanée, telle que l'eczéma.

10. Utilisation selon la revendication 9, pour la fabrication d'une composition destinée au traitement du psoriasis.

## Claims

1. Composition comprising, in a pharmaceutically acceptable vehicle:
a) between 0.0001 and 0.1% of clobetasol propionate in solubilized form;
b) between 0.00001 and 0.1% of calcitrol in solubilized form;
c) between 5 and 60% of ethanol;
d) between 10 and 60% of hexamethyldisiloxane; and
e) between 1 and 80% of a non-volatile oily phase composed of caprylic/capric triglycerides;
the composition being liquid at ambient temperature and sprayable.

2. Composition according to Claim 1 comprising, in a pharmaceutically acceptable vehicle:
a) between 0.001 and 0.05% of clobetasol propionate;
b) between 0.0002 and 0.0005% of calcitriol;
c) between 10 and 40% of ethanol;
d) between 15 and 45% of hexamethyldisiloxane; and
e) between 30 and 45% of an oily phase composed of caprylic/capric triglycerides.

3. Composition according to either one of Claims 1 and 2, **characterized in that** it also comprises an antioxidant compound.

4. Composition according to any one of Claims 1 to 3, **characterized in that** the antioxidant is selected from DL-α-tocopherol, butylhydroxyanisole and butylhydroxytoluene.

5. Composition according to any one of Claims 1 to 4, **characterized in that** it also comprises a silicone gum.

6. Composition according to any one of Claims 1 to 5, **characterized in that** it also comprises a surfactant compound.

7. Composition according to Claim 6, **characterized in that** the surfactant is selected from sodium laurylsulphate, poloxamers and polysorbates.

8. Composition according to one of Claims 1 to 7, **characterized in that** it comprises a propenetrating agent.

9. Use of a composition according to any one of Claims 1 to 8 for the preparation of a drug intended for the treatment of:
- dermatological complaints with an inflammatory immunoallergic component and with or without cellular proliferation disorder, especially cutaneous, mucous or ungueal psoriasis, psoriatic rheumatism, and cutaneous atopy such as eczema.

10. Use according to Claim 9 for the preparation of a composition intended for the treatment of psoriasis.

## Patentansprüche

1. Zusammensetzung, die in einem pharmazeutisch akzeptablen Träger enthält:
a) 0,0001 bis 0,1 % Clobetasolpropionat in solubilisierter Form;
b) 0,00001 bis 0,1 % Calcitriol in solubilisierter Form;
c) 5 bis 60 % Ethanol;
d) 10 bis 60 % Hexamethyldisiloxan;
e) 1 bis 80 % nichtflüchtige Ölphase, die aus Capryl/Caprinsäure-Triglyceriden zusammengesetzt ist;
wobei die Zusammensetzung bei Raumtemperatur flüssig und zerstäubbar ist.

2. Zusammensetzung nach Anspruch 1, die in einem pharmazeutisch akzeptablen Träger enthält:
a) 0,001 bis 0,05 % Clobetasolpropionat;
b) 0,0002 bis 0,0005 % Calcitriol;
c) 10 bis 40 % Ethanol;
d) 15 bis 45 % Hexamethyldisiloxan;
e) 30 bis 45 % Ölphase, die aus Capryl/Caprinsäure-Triglyceriden zusammengesetzt ist.

3. Zusammensetzung nach einem der Ansprüche 1 bis 2, **dadurch gekennzeichnet, dass** sie auch ein Antioxidationsmittel enthält.

4. Zusammensetzung nach einem der Ansprüche 1 bis 3, **dadurch gekennzeichnet, dass** das Antioxidationsmittel unter DL-α-Tocopherol, Butylhydroxyanisol und Butylhydroxytoluol ausgewählt ist.

5. Zusammensetzung nach einem der Ansprüche 1 bis 4, **dadurch gekennzeichnet, dass** sie auch einen Silicongummi enthält.

6. Zusammensetzung nach einem der Ansprüche 1 bis 5, **dadurch gekennzeichnet, dass** sie ferner einen grenzflächenaktiven Stoff enthält.

7. Zusammensetzung nach Anspruch 6, **dadurch gekennzeichnet, dass** der grenzflächenaktive Stoff unter Natriumlaurylsulfat, Poloxameren und Polysorbaten ausgewählt ist.

8. Zusammensetzung nach einem der Ansprüche 1 bis 7, **dadurch gekennzeichnet, dass** sie einen penetrationsfördernden Stoff enthält.

9. Verwendung einer Zusammensetzung nach einem der Ansprüche 1 bis 8 für die Herstellung eines Arzneimittels, das für die Behandlung der folgenden Erkrankungen vorgesehen ist:
- dermatologischen Erkrankungen mit einer entzündlichen immunoallergischen Komponente, mit oder ohne Störung der Zellproliferation, insbesondere Psoriasis der Haut, der Schleimhäute oder der Nägel, Psoriasis arthropathica, Atopie der Haut, wie Ekzemen.

10. Verwendung nach Anspruch 9 für die Herstellung einer Zusammensetzung, die zur Behandlung von Psoriasis vorgesehen ist.
